# EUROPEAN PATENT SPECIFICATION

(11) **EP 3 531 109 B1**
(45) Date of publication and mention of the grant of the patent: **18.11.2026**
(21) Application number: 19157847.5
(22) Date of filing: 18.02.2019
(51) Int. Cl.: G01N 21/3563, G01N 21/63, G01N 21/88, G01N 33/00

(54) **ACTIVE REAL-TIME CHARACTERIZATION SYSTEM PROVIDING SPECTRALLY BROADBAND CHARACTERIZATION**
SYSTEM ZUR AKTIVEN ECHTZEITCHARAKTERISIERUNG MIT BEREITSTELLUNG VON SPEKTRAL BREITBANDIGER CHARAKTERISIERUNG
SYSTÈME DE CARACTÉRISATION ACTIVE EN TEMPS RÉEL FOURNISSANT UNE CARACTÉRISATION SPECTRALE À LARGE BANDE

(30) Priority: 22.02.2018 US 201815902201
(43) Date of publication of application: 28.08.2019
(73) Proprietor: The Boeing Company, Arlington, VA 22202 (US)
(72) Inventor: HUNT, Jeffrey H., Chicago, IL 60606-1596 (US)
(74) Representative: Morrall, Jonathan Ian McLachlan

(56) References cited:
- EP-A1- 2 977 747
- US-A1- 2015 041 654
- US-A1- 2018 013 964
- US-B2- 9 404 854
- VARAGNAT ANTOINE: "Second-Harmonic Generation (SHG) for Studies of Surfaces and Interfaces", 12 October 2020 (2020-10-12), pages 1 - 4, XP055869994, Retrieved from the Internet <URL:https://andor.oxinst.com/learning/view/article/second-harmonic-generation-(shg)-for-studies-of-surfaces-and-interfaces> [retrieved on 20211207]

## Description

### FIELD

This disclosure relates generally to an active real-time characterization system that provides spectrally broadband characterization.

### BACKGROUND

In recent years, aircraft manufacturers have developed aircraft designs and aircraft fabrication methods that make greater use of carbon fiber composite materials and the like ("composite materials" or "CFCM"), such as graphite/epoxy, carbon fiber reinforced plastic ("CFRP") and graphite reinforced polymer ("GRP"). Composite materials are significantly lighter than traditional aircraft materials (e.g. aluminum, titanium, steel and alloys thereof), and can provide high strength with low weight, allowing lighter, more fuel efficient aircraft. In some newer aircraft, for example, the majority of the primary structure, including the fuselage and wing, is made of composite materials. One drawback in the growing use of carbon fiber composite materials is the lack of effective non-destructive evaluation type testing methodologies available for testing such materials during and after manufacture. In particular, there is a lack of non-destructive evaluation type testing systems for providing real time characterization of composites during overlay manufacturing.

Accordingly, there is a need for a testing system which addresses the drawbacks identified above.

US 2018/013964 A1 describes a system for providing active real-time characterization of an article under test. An infrared light source, a first visible light source and a second visible light source each outputs and directs a beam of coherent light at a particular area on the article under test. A visible light camera and a visible light second harmonic generation camera, an infrared camera and an infrared second harmonic generation camera, a sum frequency camera and a third order camera are each configured to receive a respective predetermined return beam of light from the particular area on the article under test. A processor receives signals from the cameras and calculates in real time respective spectroscopic signals and compares each calculated signal with each other calculated signal and with a predetermined baseline signal to ensure that the article under test conforms to an expected value.

EP 2 977 747 A1 describes a method and apparatus for testing materials. More particularly, but not exclusively, this disclosure concerns a method and apparatus for testing materials using infrared spectrometry. The disclosure also concerns the calibration of an infrared spectrometer for use in testing materials using infrared spectrometry. The disclosure provides a method of calibrating an infrared spectrometer for testing composites in the aerospace industry, comprising the steps of, selecting a plurality of variables which have the potential to influence the physical characteristics of a composite used in the aerospace industry, selecting a plurality of values for each variable and inputting the variable and values into a design of experiments model, thereby obtaining a sample test matrix.

US 2015/041654 A1 describes a method for non-destructive, contact or non-contact inspection of composite assemblies using radiation having a frequency in the terahertz range (10 GHz-10 THz) of the spectrum, whereby said method is implemented as an embodiment of the system for non-destructive, contact or non-contact inspection of composite assemblies using terahertz radiation, that is also under the disclosure. Said method enables the forming of a two or three-dimensional image of the material structure of an assembly of composite materials, from which image detection and analysis of material conditions of the composite materials forming said composite assemblies is possible, irrespective of the way that the composite materials forming said composite assemblies were joined together, and without the need for a priori knowledge about the structural characteristics, shape or configuration of said composite materials (for instance layered, foam, placed on metal substrate).

### SUMMARY

The invention to which this European patent is directed provides a system for providing active real-time characterization of an article under test. The system includes a broadband infrared light source for outputting a beam of broadband infrared light. The infrared light source is configured to direct the beam of broadband infrared light at a particular area on the article under test. A broadband IR light detector is configured to receive a first predetermined return beam of light from the particular area on the article under test. A processor is coupled to receive signals from the broadband IR light detector. The processor is configured to calculate in real time a spectrally sensitive signal, the processor configured to analyze the calculated spectrally sensitive signal to ensure that the article under test conforms to an expected value. The system includes a first visible light source configured to output a first beam of coherent visible light. The first visible light source is configured to direct the first beam of coherent visible light at the same particular area on the article under test. A visible light camera and a visible light second harmonic generation camera are each configured to receive a second predetermined return beam of light from the particular area on the article under test. An infrared camera and an infrared second harmonic generation camera are each configured to receive a third predetermined return beam of light from the particular area on the article under test. The first visible light source and the broadband IR light source are configured and positioned to provide light signals which allow the processor to generate simultaneous linear and second harmonic generation real time spectroscopic signals, in conjunction with the visible light camera and the visible light second harmonic generation camera and the infrared camera and the infrared second harmonic generation camera. The processor is coupled to receive signals from the visible light camera, the visible light second harmonic generation camera, the infrared camera and the infrared second harmonic generation camera. The processor is configured to calculate in real time a linear spectroscopic signal and a second harmonic generation spectroscopic signal. The processor is configured to compare each calculated signal with each other calculated signal and with a predetermined baseline signal to ensure that the article under test conforms to an expected value.

In an embodiment of the invention to which this European patent is directed, the system further includes a second visible light source for outputting a second beam of coherent visible light. The second visible light source is configured to direct the second beam of coherent visible light at the same particular area on the article under test. A sum-frequency camera is configured to receive a fourth return beam of light from the particular area on the article under test. A third-order camera is configured to receive a fifth return beam of light from the particular area on the article under test. The processor is 'coupled to receive signals from the sum-frequency camera and the third-order camera. The processor is configured to calculate in real time a sum-frequency spectroscopic signal and a third-order spectroscopic signal and to compare the sum-frequency spectroscopic signal and the third-order spectroscopic signal with the other calculated signals and with a predetermined baseline signal to ensure that the article under test conforms to an expected value.

In an embodiment of the invention to which this European patent is directed, each of the sources includes an intensity control for setting a predetermined intensity for the respective output beam of light and/or a polarization control for setting a predetermined polarization for the respective output beam of light. Similarly, in an embodiment of the invention, to which this European patent is directed, each of the cameras includes an intensity control for setting a predetermined intensity for the respective input beam of light and/or a polarization control for setting a predetermined polarization for the respective input beam of light. In an embodiment of the invention to which this European patent is directed, a beam splitter is configured to split a return beam of light into two portions, a first portion directed to the visible light camera and a second portion directed to the visible light second harmonic generation camera. Finally, in an embodiment of the invention to which this European patent is directed the broadband IR light detector may be a broadband IR camera or a spectrometer.

In a second aspect, not forming part of the claimed invention to which this European patent is directed, a system for providing active real-time characterization of an article under test is provided.

The system includes a broadband infrared light source for outputting a beam of broadband infrared light, the infrared light source configured to direct the beam of broadband infrared light at a particular area on the article under test. A first visible light source outputs a first beam of coherent visible light. The first visible light source is configured to direct the first beam of coherent visible light at the same particular area on the article under test. A second visible light source outputs a second beam of coherent visible light. The second visible light source is configured to direct the second beam of coherent visible light at the same particular area on the article under test. A broadband IR light detector is configured to receive a first predetermined return beam of light from the particular area on the article under test. A third-order camera is configured to receive a third return beam of light from the particular area on the article under test. A processor is coupled to receive signals from the broadband IR light detector, and the third-order camera. The processor is configured to calculate in real time a spectrally sensitive signal and a third-order spectroscopic signal. The processor is configured to analyze the calculated spectrally sensitive signal to ensure that the article under test conforms to an expected value and to compare each calculated signal with each other calculated signal and with a predetermined baseline signal to ensure that the article under test conforms to an expected value.

In a further example of the second aspect, a visible light camera and a visible light second harmonic generation camera are each configured to receive a second predetermined return beam of light from the particular area on the article under test. An infrared camera and an infrared second harmonic generation camera are each configured to receive a third predetermined return beam of light from the particular area on the article under test. The processor is coupled to receive signals from the visible light camera, the visible light second harmonic generation camera, the infrared camera, and the infrared second harmonic generation camera. The processor is configured to calculate in real time a linear spectroscopic signal and a second harmonic generation spectroscopic signal and to compare the linear spectroscopic signal and the second harmonic generation spectroscopic signal with the other calculated signals and with a predetermined baseline signal to ensure that the article under test conforms to an expected value.

In a still further example of the second aspect, a sum-frequency camera is configured to receive a fourth return beam of light from the particular area on the article under test. The processor is coupled to receive signals from the sum-frequency camera. The processor is configured to calculate in real time a sum-frequency signal and to compare the sum-frequency signal with the other calculated signals and with a predetermined baseline signal to ensure that the article under test conforms to an expected value.

In a third aspect not forming part of the claimed invention to which this European patent is directed, a method for active real-time characterization of an article under test is provided. A broadband infrared light source is directed at an area on a surface of the article under test. A broadband IR light detector is aligned to receive light from the broadband infrared light source reflected from the surface of the article under test. Data is acquired from the broadband IR light detector based on the received light. The data from the broadband IR light detector is analyzed and correlations among the data are observed. Finally, the correlations among the data are analyzed and interpreted to ensure that the article under test conforms to an expected value.

In a further example of the third aspect, a first visible light source and a second visible light source is directed at the area on a surface of the article under test. A visible light camera and a visible light second harmonic generation camera are aligned to receive light from the broadband infrared light source, the first visible light source, and the second visible light source reflected from the surface of the article under test. Data is acquired from the visible light camera and the visible light second harmonic generation camera. The data from the visible light camera and the visible light second harmonic generation camera is analyzed and correlations among the data from the broadband IR light detector, the visible light camera and the visible light second harmonic generation camera are observed. The correlations among the data is analyzed and interpreted to ensure that the article under test conforms to an expected value.

The features, functions, and advantages that have been discussed can be achieved independently in various examples or may be combined in yet other examples, further details of which can be seen with reference to the following description and drawings.

### BRIEF DESCRIPTION OF THE DRAWINGS

The following detailed description, given by way of example and not intended to limit the present disclosure solely thereto, will best be understood in conjunction with the accompanying drawings in which:
FIG. 1 is a block diagram of an active real-time characterization system for use during composite overlay manufacturing according to an embodiment of the present invention to which this European patent is directed;
FIG. 2 is a block diagram showing the composition of the infrared light and visible light camera systems that can be used in embodiments of the present invention to which this European patent is directed;
FIG. 3 is a block diagram showing the composition of the light sources that can be used in embodiments of the present invention to which this European patent is directed;
FIG. 4 is a block diagram showing the composition of the cameras that can be used in embodiments of the present invention to which this European patent is directed; and
FIG. 5 is a flow chart of a method for operating the active real-time characterization system of the present disclosure.

### DETAILED DESCRIPTION

In the present disclosure, like reference numbers refer to like elements throughout the drawings.

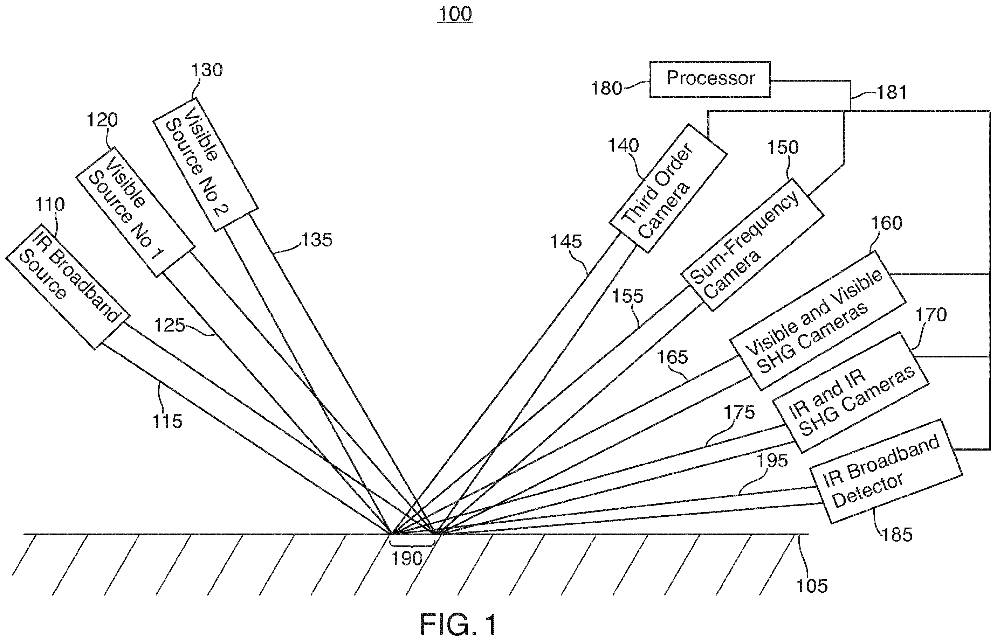

Referring now to FIG. 1, the active real-time characterization system 100 of the present disclosure includes a broadband infrared (IR) light source 110, a first visible light source 120 and a second visible light source 130. The use of broadband IR light source 110 allows system 100 to provide spectral sensitivity. Each of the light sources 110, 120, 130 is positioned to direct a beam of light at an area 190 on a surface of an article under test 105, e.g., a part constructed from carbon-fiber composites. In particular, broadband IR source 110 is positioned to direct a beam of light 115 at area 190, first visible light source 120 is positioned to direct a coherent beam of light 125 at area 190, and send visible light source 130 is positioned to direct a beam of coherent visible light 135 at area 190. The beam of light 115 and beams of coherent visible light 125, 135 are emitted directly from the respective light sources 110, 120, 130 or are emitted via respective optical fibers (and appropriate lenses, etc.) coupled to the light sources 110, 120, 130. When the beam of light 115 and beams of coherent visible light 125, 135 are emitted via respective optical fibers, each light source 110, 120 130 consists of a laser of the appropriate type (visible or IR light) that is coupled to an input of an associated optical fiber via input optics. The laser is preferably a solid state laser or a diode laser and may be, for example, a diode laser, a continuous-wave diode laser, a solid state laser, a continuous-wave solid state laser, a flash-lamp pumped solid state laser, or a diode pumped solid state laser. The input optics consist of an input polarizer, an input wavelength discriminator, an input spatial filter and an input propagation optics. The input polarizer is, for example, a Brewster angle polarizer, a thin film polarizer, a Glan-air or Glan-Thompson polarizer or other crystal polarizer. The wavelength discriminator is, for example, a color filter, a dielectric film, a holographic transmission filter, or a grating. The input propagation optics is formed of one or more refractive or reflective optics which, when used in combination, control the divergence or convergence of the beam as it propagates towards the first input optical fiber. The input optics are optimized for the wavelength of the associated optical source. Coupling optics are coupled to an output of each optical fiber to direct the beam to area 190. Broadband IR light source 110 is configured to output light at a broad spectrum of IR wavelength (i.e., a spectrum of IR radiation frequencies that excite or interact with multiple IR resonances or material responses at the same time), while first visible light source 120 is configured to output light at a first fixed, predetermined visible wavelength and second visible light source 130 is configured to output light at a second fixed, predetermined visible wavelength, different from the first fixed, predetermined visible wavelength.

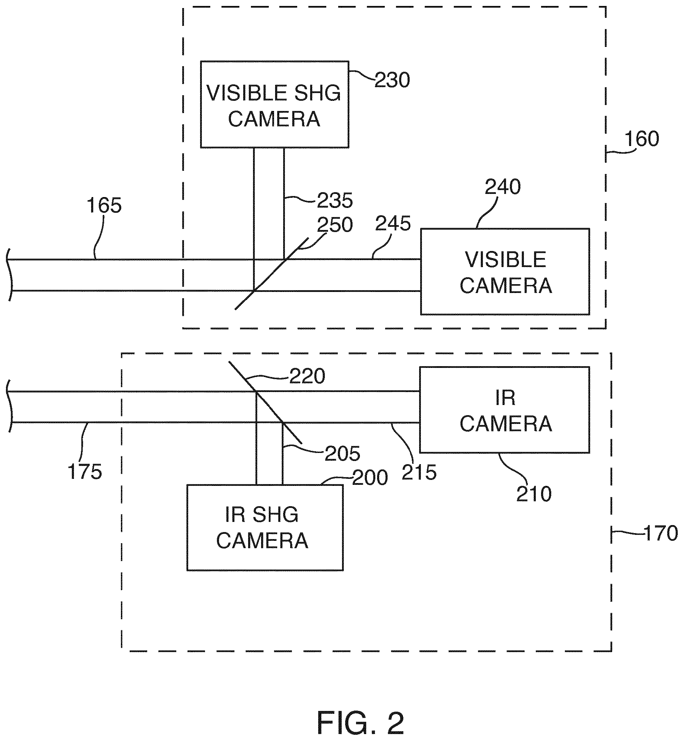

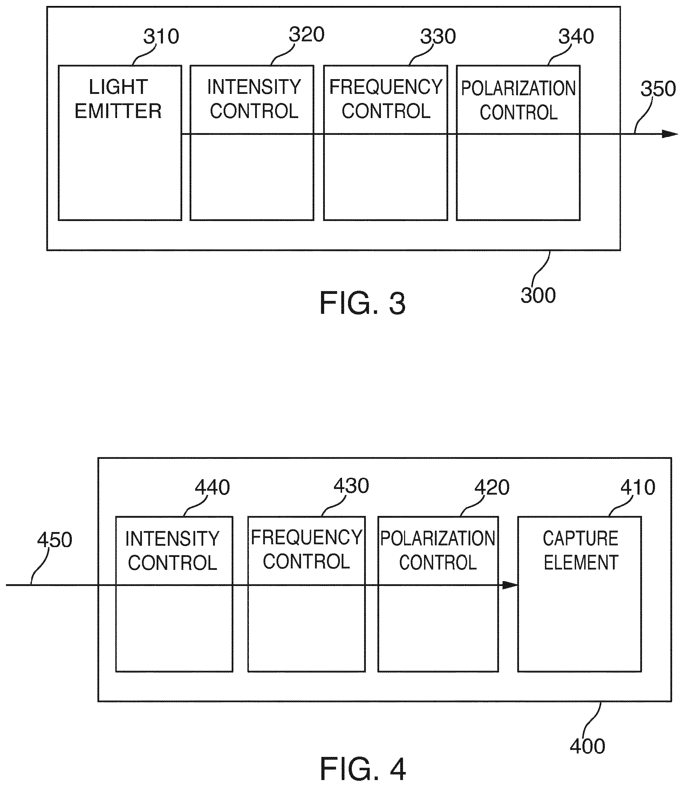

System 100 in FIG. 1 also includes a number of cameras or detectors for detecting light reflected from the surface of an article under test 105, including a Raman (third-order) camera 140 which receives a light beam 145 and a sum-frequency camera 150 which receives a light beam 155. System 100 also includes paired visible and visible second harmonic generation (SHG) cameras 160 which receive a light beam 165, and paired IR and IR SHG cameras 170 which receive a light beam 175. Finally, system 100 includes a broadband IR light detector 185 which receives a light beam 195. Detector 185 may be a broadband IR camera or a spectrometer, for example. Referring now to FIG. 2, the paired visible light and visible light second harmonic generation (SHG) cameras 160 include a visible light SHG camera 230 and a visible light camera 240 which are positioned to each receive light beam 165 via a beam splitter 250. In particular, beam splitter 250 is positioned to split light beam 165 into a first portion 235 that is provided to visible light SHG camera 230 and a second portion 245 that is provided to visible light camera 240. Similarly, the paired IR and IR second harmonic generation (SHG) cameras 170 include an IR SHG camera 200 and an IR camera 210 which are positioned to each receive light beam 175 via a beam splitter 220. In particular, beam splitter 220 is positioned to split light beam 175 into a first portion 205 that is provided to IR SHG camera 200 and a second portion 215 that is provided to IR camera 210. Each of the cameras/detectors 140, 150, 190, 200, 210, 230 and 240 produces an output signal that is communicated in a conventional manner to a processor 180 in FIG. 1 via a link 181 for processing as discussed below. As also discussed below, the reflected light beams 145, 155, 165, 175, and 195 are at a particular angle with respect to the surface of an article (sometimes referred to herein as a "device") under test 105 based on the fixed angles that the beam of light 115 and beams of coherent visible light 125 and 135 are directed at the surface of the article ("device") under test 105. The cameras/detectors 140, 150, 190, 200, 210, 230 and 240 are thus positioned to receive such light beams. Each camera 140, 150, 200, 210, 230, 240 is a conventional detector as defined below with respect to FIG. 4.

As one of ordinary skill in the art will readily recognize, light sources 110, 120, 130 and cameras/detectors 140, 150, 190, 200, 210, 230 and 240 are fixed in place and the surface of the article under test 105 may be moved so that the area 190 of the beam of light 115 and beams of coherent visible light 125, 135 moves over the entire surface of article under test 105. In another example, light sources 110, 120, 130 and cameras 140, 150, 190, 200, 210, 230 and 240 are mounted on a fixture that moves along the surface of article under test 105. In yet another example, light sources 110, 120, 130 are arranged to raster the respective output beam of light 115 and beams of coherent visible light 125, 135 across the surface of the article under test 105, and the cameras/detectors 140, 150, 190, 200, 210, 230 and 240 arranged to move proportionally to receive the respective associated return light beams 145, 155, 165, 175, 195.

Referring now to FIG. 3, each of the light sources 120, 130 is configured similarly, as shown by representative light source 300, but each is configured for a different intensity, frequency and/or polarization. Light source 110 is a broadband IR light source that does not require any frequency control but may include intensity and frequency controls similar to that of representative light source 300. Light source 300 includes a light emitter 310, an intensity control 320, a frequency control 330 and a polarization control 340 for outputting a light beam 350. Light emitter 310 is preferably a narrow frequency bandwidth visible pulse laser and, may be, for example a pulsed diode laser, a continuous wave diode laser or a pulsed solid state laser or a continuous wave solid state laser. Intensity controls may include broadband filters for reducing intensity or may specifically refer to certain frequency notch filters which are intended to drop intensity levels where the cameras/detectors can act in a linear fashion. Frequency controls can be accomplished in simple cases by frequency dependent color filters or notch filters and in more elaborate by a spectrophotometer that is typically composed of a diffraction grating which operates at a frequency or bandwidth of interest. The key point for the frequency control is to ensure that only light in beam 350 is directed at the surface of the article under test 105 and that stray light produced by light emitter 310 is removed, and as one of ordinary skill in the art will readily recognize, other frequency selective elements may also be used. Polarization control typically consists of two separate optical elements, a polarizer which only passes light of one polarization and a polarization modifying element - typically a halfway plate or a quarter wave plate. A halfway plate is used to rotate the polarization to the desired orientation. A quarter wave plate is used to change the polarization from linear to circular or from circular to linear as needed. As shown, the polarizer is the last element before light beam 350 leaves the source and heads for the surface of the article under test105. Each light source 110, 120, 130 is configured, based on the selection of light emitter 310, intensity control 320, frequency control 330 and polarization control 340 in each to provide a respective beam of light 115 and beam of coherent visible light 125, 135.

Referring now to FIG. 4, each of the cameras 140, 150, 200, 210, 230 and 240 is configured similarly, as shown by representative camera 400. Detector 185 is a broadband IR camera or a spectrometer and does not require any frequency control but includes intensity and frequency controls similar to that of representative camera 400. Camera 400 includes a capture element 410, a polarization control 420, a frequency control 430 and an intensity control 440 for receiving light beam 450. Exemplary capture elements 34 include, without limitation, cameras, CCD devices, imaging arrays, photometers, and like devices. The frequency control 430 and intensity control 440 operate on light beam 450 in a similar manner as intensity control 320 and frequency control 330 operate on light beam 350 as discussed above. Preferably, polarization control 420 consists of a half wave plate and quarter wave combination, followed by a polarizer.

In operation, the system 100 shown in FIG. 1 provides a combination of linear infrared spectroscopy, second order surface frequency mixing spectroscopy, and third-order nonlinear optics (e.g., Raman spectroscopy) spectroscopy. In addition, the use of a broadband IR light source and corresponding broadband IR light detector allows processor 180 to generate a spectrally sensitive signal. Processor 180 analyzes this spectrally sensitive signal to identify peaks thereon. A signal based on a broad IR input will naturally have some IR frequencies that are resonant with materials present at the surface or interface of the article under test 105. The presence of a resonance leads to enhanced nonlinear interaction, which results in such "peaks" in the output spectrum. Processor 180 is configured to interrogate the output spectrum of the spectrally sensitive signal in order to determine the presence of any peaks. System 100 provides a number of ways of performing species identification and allows the cross correlation between the three types of spectroscopies in order to avoid false negative spectral features.

In particular, visible light source 120 and broadband IR light source 110 are configured and positioned to provide light signals which allow the processor 180 to generate simultaneous linear (same frequency) and non-linear (second harmonic generation) real time spectroscopic signals, in conjunction with paired visible light and visible light second harmonic generation (SHG) cameras 160 and paired IR and IR SHG cameras 170. As one of ordinary skill in the art will readily recognize, paired visible light and visible light second harmonic generation (SHG) cameras 160 and paired IR and IR SHG cameras 170 are positioned at a particular predetermined angle to receive the appropriate respective return light beams 165, 175 from surface of the article under test 105.

Further, visible light source 120 and broadband IR light source 110 are also configured and positioned to provide light signals which allow the processor 180 to generate a sum-frequency (ω_{IR} + ω_{VISIBLE}) real-time spectroscopic signal, in conjunction with sum-frequency camera 150. As one of ordinary skill in the art will readily recognize, sum-frequency camera 150 is positioned at a particular predetermined angle to receive the appropriate return light beams 155 from the surface of the article under test 105.

Finally, visible light source 120 and visible light source 130 are configured and positioned to provide light signals which allow the processor 180 to generate a third-order (2ω_{VIS1} - ω_{VIS2}) (e.g., Raman) real-time spectroscopic signal, in conjunction with Raman (third-order) camera 140. As one of ordinary skill in the art will readily recognize, Raman (third-order) camera 140 is positioned at a particular predetermined angle to receive the appropriate return light beam 145 from the surface of the article under test 105.

The processor 180 is coupled to receive signals from each of cameras/detectors 140, 150, 190, 200, 210, 230 and 240 and is configured to calculate in real time a linear spectroscopic signal, a second harmonic generation spectroscopic signal, a sum-frequency spectroscopic signal and a third-order spectroscopic signal, and a spectrally sensitive signal. The processor 180 is also configured to compare each calculated signal with each other calculated signal and with a predetermined baseline signal to ensure that the article under test conforms to an expected value. Processor 180 also processes the spectrally sensitive signal in order to identify any peaks therein. When the processor 180 determines that the calculated signals indicate that the article under test does not conform to the expected value, processor 180 provides a fault signal which is used to halt formation of the part under test 105 for either repair thereof or so that part under test 105 may be immediately discarded.

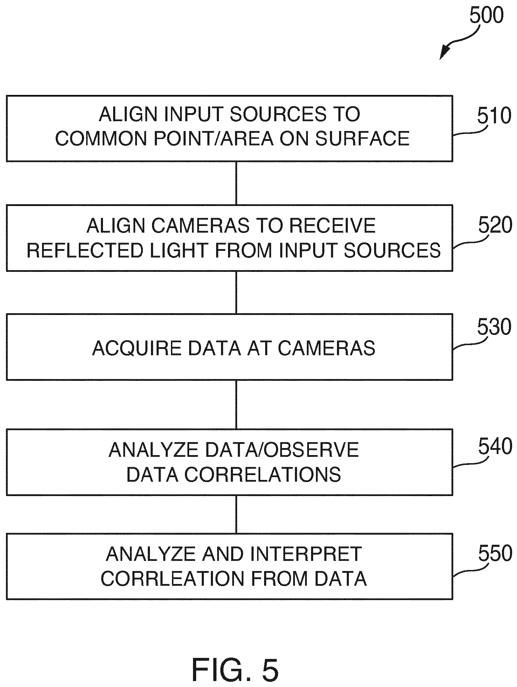

Referring now to FIG. 5, a flow chart 500 of a method for operating the active real-time characterization system of the present disclosure is shown. In step 510, the input light sources are aligned to a common point or area on the surface of the article under test. Next, at step 520, the cameras are aligned to receive light reflected from the input sources. As evident from the discussion above, the third-order camera 140, the sum-frequency camera 150, the visible light and visible light SHG cameras 160, the IR and IR SHG cameras 170, and the broadband IR light detector 185 are aligned separately. Further, at step 530, data is acquired at each of the cameras/detectors. After this, at step 540, the acquired data is analyzed, and correlations among the data are observed. Finally, at step 550, the correlations among the data are interpreted to ensure that the article under test conforms to an expected value.

As one of ordinary skill in the art will readily recognize, the active-real time characterization system 100 of the present disclosure may be applied to testing for composition of the article under test, for identifying contamination on the surface of the article under test or for a combination of both material composition and contamination identification. In addition, the active real-time characterization system 100 of the present disclosure may be used to test graphite fiber-based materials, polymer/plastic materials, glass fiber reinforcement-based materials such as fiberglass and glass laminate aluminum reinforced epoxy ("GLARE"), resins, plastics or polymers without reinforcement fibers, metals, and ceramics including glass materials.

By providing a combination of linear, non-linear, sum-frequency and third-order real time spectroscopic signals, the present system provides the ability to more accurately monitor the chemistry of composite parts during formation by avoiding false negative spectral features. This system can allow, in some cases, defects to be repaired and thus prevent the loss of the composite part. In other cases, this system can ensure that production is halted as soon as a defect is identified, and thus preventing further costly work from being performed on a composite part destined to be discarded.

In further examples, the data generated by the active-real time characterization system 100 of the present disclosure is combined for analysis with data separately generated by way of UV-VIS-NIR spectroscopy, IR spectroscopy, microwave spectroscopy, THz spectroscopy, ultrasonic NDE/NDI, Raman spectroscopy, Brillouin spectroscopy and/or ellipsometry.

Although the present disclosure has been particularly shown and described with reference to the preferred examples and various aspects thereof, it will be appreciated by those of ordinary skill in the art that various changes and modifications may be made without departing from the scope of the disclosure.

## Claims

1. A system for providing active real-time characterization of an article under test, comprising:
a broadband infrared "IR" light source (110) for outputting a beam of broadband infrared light (115), the broadband infrared light source configured to direct the beam of broadband infrared light at a particular area (190) on the article under test;
a broadband IR light detector (185) configured to receive a first predetermined return beam of light (195) from the particular area on the article under test;
a processor (180) coupled to receive signals from the broadband IR light detector, the processor configured to calculate in real time a spectrally sensitive signal, the processor configured to analyze the calculated spectrally sensitive signal to ensure that the article under test conforms to an expected value;
a first visible light source (120) for outputting a first beam of coherent visible light (125), the first visible light source configured to direct the first beam of coherent visible light at the same particular area on the article under test;
a visible light camera (240) and a visible light second harmonic generation camera (230), the visible light camera and visible light second harmonic generation camera each configured to receive a second predetermined return beam of light (165) from the particular area on the article under test;
an infrared camera (210) and an infrared second harmonic generation camera (200), the infrared camera and infrared second harmonic generation camera each configured to receive a third predetermined return beam of light (175) from the particular area on the article under test;
wherein the first visible light source (120) and the broadband IR light source (110) are configured and positioned to provide light signals which allow the processor (180) to generate simultaneous linear and second harmonic generation real time spectroscopic signals, in conjunction with the visible light camera (240) and the visible light second harmonic generation camera (230) and the infrared camera (210) and the infrared second harmonic generation camera (200); and
wherein the processor is coupled to receive signals from the visible light camera, the visible light second harmonic generation camera, the infrared camera and the infrared second harmonic generation camera, and is configured to calculate in real time a linear spectroscopic signal and a second harmonic generation spectroscopic signal, the processor configured to compare each calculated signal with each other calculated signal and with a predetermined baseline signal to ensure that the article under test conforms to an expected value.

2. The system of claim 1, further comprising:
a second visible light source (130) for outputting a second beam of coherent visible light (135), the second visible light source configured to direct the second beam of coherent visible light at the same particular area on the article under test;
a sum-frequency camera (150) configured to receive a fourth return beam of light (155) from the particular area on the article under test;
a third-order camera (140) configured to receive a fifth return beam of light (145) from the particular area on the article under test; and
wherein the processor is coupled to receive signals from the sum-frequency camera and the third-order camera, and the processor is configured to calculate in real time a sum-frequency spectroscopic signal and a third-order spectroscopic signal and to compare the sum-frequency spectroscopic signal and the third-order spectroscopic signal with the other calculated signals and with a predetermined baseline signal to ensure that the article under test conforms to an expected value.

3. The system of any of claims 1-2, wherein each of the sources includes an intensity control (320) for setting a predetermined intensity for the respective output beam of light.

4. The system of any of claims 1-3, wherein each of the sources includes a polarization control (340) for setting a predetermined polarization for the respective output beam of light.

5. The system of any of claims 1-4, wherein each of the cameras includes an intensity control (440) for setting a predetermined intensity for the respective input beam of light.

6. The system of any of claims 1-5, wherein each of the cameras includes a polarization control (420) for setting a predetermined polarization for the respective input beam of light.

7. The system of any of claims 1-6, further comprising a beam splitter (250) configured to split a return beam of light into two portions, a first portion directed to the visible light camera and a second portion directed to the visible light second harmonic generation camera.

8. The system of any of claims 1-7, wherein the broadband IR light detector is a broadband IR camera or a spectrometer.

## Patentansprüche

1. System zum Bereitstellen einer aktiven Echtzeitcharakterisierung eines zu prüfenden Artikels, welches Folgendes umfasst:
eine Breitband-Infrarot- bzw. -"IR"-Lichtquelle (110) zum Ausgeben eines Strahls von Breitbandinfrarotlicht (115), wobei die Breitbandinfrarotlichtquelle dazu konfiguriert ist, den Strahl von Breitbandinfrarotlicht auf einen bestimmten Bereich (190) auf dem zu prüfenden Artikel zu richten;
einen Breitband-IR-Lichtdetektor (185), der dazu konfiguriert ist, einen ersten vorbestimmten zurückkehrenden Lichtstrahl (195) von dem bestimmten Bereich auf dem zu prüfenden Artikel zu empfangen;
einen Prozessor (180), der gekoppelt ist, um Signale von dem Breitband-IR-Lichtdetektor zu empfangen, wobei der Prozessor dazu konfiguriert ist, in Echtzeit ein spektral empfindliches Signal zu berechnen, wobei der Prozessor dazu konfiguriert ist, das berechnete spektral empfindliche Signal zu analysieren, um sicherzustellen, dass der zu testende Artikel einem erwarteten Wert entspricht;
eine erste Sichtbares-Licht-Quelle (120) zum Ausgeben eines ersten Strahls von kohärentem sichtbarem Licht (125), wobei die erste Sichtbares-Licht-Quelle dazu konfiguriert ist, den ersten Strahl von kohärentem sichtbarem Licht auf denselben bestimmten Bereich auf dem zu testenten Artikel zu richten;
eine Sichtbares-Licht-Kamera (240) und eine Sichtbares-Licht-Kamera (230) für die Erzeugung der zweiten Harmonischen, wobei die Sichtbares-Licht-Kamera und die Sichtbares-Licht-Kamera für die Erzeugung der zweiten Harmonischen jeweils dazu konfiguriert sind, einen zweiten vorbestimmten zurückkehrenden Lichtstrahl (165) von dem bestimmten Bereich auf dem zu testenden Artikel zu empfangen;
eine Infrarotkamera (210) und eine Infrarotkamera (200) für die Erzeugung der zweiten Harmonischen, wobei die Infrarotkamera und die Infrarotkamera für die Erzeugung der zweiten Harmonischen jeweils dazu konfiguriert sind, einen dritten vorbestimmten zurückkehrenden Lichtstrahl (175) von dem bestimmten Bereich auf dem zu testenden Artikel zu empfangen;
wobei die erste Sichtbares-Licht-Quelle (120) und die Breitband-IR-Lichtquelle (110) dazu konfiguriert und positioniert sind, Lichtsignale bereitzustellen, die es dem Prozessor (180) ermöglichen, simultane lineare Echtzeitspektroskopiesignale und Echtzeitspektroskopiesignale der Erzeugung der zweiten Harmonischen in Verbindung mit der Sichtbares-Licht-Kamera (240) und der Sichtbares-Licht-Kamera (230) für die Erzeugung der zweiten Harmonischen und der Infrarotkamera (210) und der Kamera (200) für die Erzeugung der zweiten Harmonischen zu erzeugen; und
wobei der Prozessor gekoppelt ist, um Signale von der Sichtbares-Licht-Kamera, der Sichtbares-Licht-Kamera für die Erzeugung der zweiten Harmonischen, der Infrarotkamera und der Infrarotkamera für die Erzeugung der zweiten Harmonischen zu empfangen, und dazu konfiguriert ist, in Echtzeit ein lineares Spektroskopiesignal und ein Spektroskopiesignal der Erzeugung der zweiten Harmonischen zu berechnen, wobei der Prozessor dazu konfiguriert ist, jedes berechnete Signal mit jedem anderen berechneten Signal und mit einem vorbestimmten Basisliniensignal zu vergleichen, um sicherzustellen, dass der zu testende Artikel einem erwarteten Wert entspricht.

2. System nach Anspruch 1, das ferner Folgendes umfasst:
eine zweite Sichtbares-Licht-Quelle (130) zum Ausgeben eines zweiten Strahls von kohärentem sichtbarem Licht (135), wobei die zweite Sichtbares-Licht-Quelle dazu konfiguriert ist, den zweiten Strahl von kohärentem sichtbarem Licht auf denselben bestimmten Bereich auf dem zu testenten Artikel zu richten;
eine Summenfrequenzkamera (150), die dazu konfiguriert ist, einen vierten zurückkehrenden Lichtstrahl (155) von dem bestimmten Bereich auf dem zu testenden Artikel zu empfangen;
eine Kamera (140) dritter Ordnung, die dazu konfiguriert ist, einen fünften zurückkehrenden Lichtstrahl (145) von dem bestimmten Bereich auf dem zu testenden Artikel zu empfangen; und
wobei der Prozessor gekoppelt ist, um Signale von der Summenfrequenzkamera und der Kamera dritter Ordnung zu empfangen, und der Prozessor dazu konfiguriert ist, in Echtzeit ein Summenfrequenzspektroskopiesignal und ein Spektroskopiesignal dritter Ordnung zu berechnen und das Summenfrequenzspektroskopiesignal und das Spektroskopiesignal dritter Ordnung mit den anderen berechneten Signalen und mit einem vorbestimmten Basisliniensignal zu vergleichen, um sicherzustellen, dass der zu testende Artikel einem erwarteten Wert entspricht.

3. System nach einem der Ansprüche 1-2, wobei jede der Quellen eine Intensitätssteuerung (320) zum Einstellen einer vorbestimmten Intensität für den jeweiligen Ausgabelichtstrahl beinhaltet.

4. System nach einem der Ansprüche 1-3, wobei jede der Quellen eine Polarisationssteuerung (340) zum Einstellen einer vorbestimmten Polarisation für den jeweiligen Ausgabelichtstrahl beinhaltet.

5. System nach einem der Ansprüche 1-4, wobei jede der Kameras eine Intensitätssteuerung (440) zum Einstellen einer vorbestimmten Intensität für den jeweiligen Eingabelichtstrahl beinhaltet.

6. System nach einem der Ansprüche 1-5, wobei jede der Kameras eine Polarisationssteuerung (420) zum Einstellen einer vorbestimmten Polarisation für den jeweiligen Eingabelichtstrahl beinhaltet.

7. System nach einem der Ansprüche 1-6, das ferner einen Strahlteiler (250) umfasst, der dazu konfiguriert ist, einen zurückkehrenden Lichtstrahl in zwei Teile aufzuteilen, einen ersten Teil, der auf die Sichtbares-Licht-Kamera gerichtet ist, und einen zweiten Teil, der auf die Sichtbares-Licht-Kamera für die Erzeugung der zweiten Harmonischen gerichtet ist.

8. System nach einem der Ansprüche 1-7, wobei der Breitband-IR-Lichtdetektor eine Breitband-IR-Kamera oder ein Spektrometer ist.

## Revendications

1. Système destiné à assurer la caractérisation active en temps réel d'un article à tester, comprenant :
une source de lumière infrarouge « IR » à large bande (110) pour émettre un faisceau de lumière infrarouge à large bande (115), la source de lumière infrarouge à large bande étant conçue pour diriger le faisceau de lumière infrarouge à large bande vers une zone particulière (190) sur l'article à tester ;
un détecteur de lumière IR à large bande (185) conçu pour recevoir un premier faisceau lumière renvoyé prédéterminé (195) provenant de la zone particulière sur l'article à tester ;
un processeur (180) couplé pour recevoir des signaux provenant du détecteur de lumière IR à large bande, le processeur étant conçu pour calculer en temps réel un signal sensible spectralement, le processeur étant conçu pour analyser le signal sensible spectralement calculé afin de s'assurer que l'article à tester respecte une valeur prévue ;
une première source de lumière visible (120) pour émettre un premier faisceau de lumière visible cohérente (125), la première source de lumière visible étant conçue pour diriger le premier faisceau de lumière visible cohérente vers la même zone particulière sur l'article à tester ;
une caméra en lumière visible (240) et une caméra en lumière visible à génération de deuxième harmonique (230), la caméra en lumière visible et la caméra en lumière visible à génération de deuxième harmonique étant chacune conçues pour recevoir un deuxième faisceau de lumière renvoyé prédéterminé (165) provenant de la zone particulière sur l'article à tester ;
une caméra infrarouge (210) et une caméra infrarouge à génération de deuxième harmonique (200), la caméra infrarouge et la caméra infrarouge à génération de deuxième harmonique étant chacune conçues pour recevoir un troisième faisceau de lumière renvoyé prédéterminé (175) provenant de la zone particulière sur l'article à tester ;
dans lequel la première source de lumière visible (120) et la source de lumière IR à large bande (110) sont conçues et positionnées de manière à fournir des signaux lumineux qui permettent au processeur (180) de générer des signaux spectroscopiques en temps réel linéaires et de génération de deuxième harmonique simultanés, conjointement avec la caméra en lumière visible (240) et la caméra en lumière visible à génération de deuxième harmonique (230) et la caméra infrarouge (210) et la caméra infrarouge à génération de deuxième harmonique (200) ; et
dans lequel le processeur est couplé pour recevoir des signaux provenant de la caméra en lumière visible, de la caméra en lumière visible à génération de deuxième harmonique, de la caméra infrarouge et de la caméra infrarouge à génération de deuxième harmonique, et est conçu pour calculer en temps réel un signal spectroscopique linéaire et un signal spectroscopique de génération de deuxième harmonique, le processeur étant conçu pour comparer chaque signal calculé à chaque autre signal calculé et à un signal de référence prédéterminé pour s'assurer que l'article à tester respecte une valeur prévue.

2. Système de la revendication 1, comprenant en outre :
une deuxième source de lumière visible (130) pour émettre un deuxième faisceau de lumière visible cohérente (135), la deuxième source de lumière visible étant conçue pour diriger le deuxième faisceau de lumière visible cohérente vers la même zone particulière sur l'article à tester ;
une caméra à fréquence somme (150) conçue pour recevoir un quatrième faisceau de lumière renvoyé (155) provenant de la zone particulière sur l'article à tester ;
une caméra du troisième ordre (140) conçue pour recevoir un cinquième faisceau de lumière renvoyé (145) provenant de la zone particulière sur l'article à tester ; et
dans lequel le processeur est couplé pour recevoir des signaux provenant de la caméra à fréquence somme et de la caméra du troisième ordre, et le processeur est conçu pour calculer en temps réel un signal spectroscopique de fréquence somme et un signal spectroscopique du troisième ordre et pour comparer le signal spectroscopique de fréquence somme et le signal spectroscopique du troisième ordre aux autres signaux calculés et à un signal de référence prédéterminé afin de s'assurer que l'article à tester respecte une valeur prévue.

3. Système de l'une quelconque des revendications 1 et 2, dans lequel chacune des sources comporte une commande d'intensité (320) pour établir une intensité prédéterminée pour le faisceau de lumière émis respectif.

4. Système de l'une quelconque des revendications 1 à 3, dans lequel chacune des sources comporte une commande de polarisation (340) pour établir une polarisation prédéterminée pour le faisceau de lumière émis respectif.

5. Système de l'une quelconque des revendications 1 à 4, dans lequel chacune des caméras comporte une commande d'intensité (440) pour établir une intensité prédéterminée pour le faisceau de lumière d'entrée respectif.

6. Système de l'une quelconque des revendications 1 à 5, dans lequel chacune des caméras comporte une commande de polarisation (420) pour établir une polarisation prédéterminée pour le faisceau de lumière d'entrée respectif.

7. Système de l'une quelconque des revendications 1 à 6, comprenant en outre un séparateur de faisceau (250) conçu pour séparer un faisceau de lumière renvoyé en deux parties, une première partie dirigée vers la caméra en lumière visible et une deuxième partie dirigée vers la caméra en lumière visible à génération de deuxième harmonique.

8. Système de l'une quelconque des revendications 1 à 7, dans lequel le détecteur de lumière IR à large bande est une caméra IR à large bande ou un spectromètre.
